# EUROPEAN PATENT SPECIFICATION

(11) **EP 2 098 253 B1**
(45) Date of publication and mention of the grant of the patent: **22.02.2012**
(21) Application number: 09154113.6
(22) Date of filing: 02.03.2009
(51) Int. Cl.: A61L 27/08, A61L 17/08, A61L 17/00

(54) **Suture with biological material**
Wundnaht mit biologischem Material
Suture avec matériau biologique

(30) Priority: 03.03.2008 US 33246 P
(43) Date of publication of application: 09.09.2009
(73) Proprietor: Arthrex, Inc., Naples, FL 34108-1945 (US)
(72) Inventor: Dreyfuss, Peter J., Naples, FL 34112 (US); Schaneville, Tara L., Bonita Springs, FL 34135 (US)
(74) Representative: Leihkauf, Steffen Falk

(56) References cited:
- EP-A- 1 504 773
- EP-A- 1 897 500
- WO-A-01/56626
- US-A- 4 461 298
- US-A1- 2003 050 666

## Description

### FIELD OF THE INVENTION

The present invention relates to surgical suture materials and, in particular, to sutures comprising biological materials such as collagen.

### BACKGROUND OF THE INVENTION

U.S. Patent No. 4,470,941 discloses a composite suture prepared by forming a thread comprised of fibers of a first synthetic polymer, the thread further including a second synthetic polymer in intimate association with an present uniformly along the length of the first synthetic polymer, softening the second synthetic polymer to cause it to flow, and applying pressure to the softened polymer to redistribute it throughout the plurality of fibers, and into the interstices. The '941 patent, however, only relates to polymers. The present invention expands upon this by allowing for a different filler material, for example, a biological material such as collagen, to improve heating. EP 1 504 773 describes a suture with fibers of collagen braided with ultra-high molecular weight polyethelene, in which the biological material is braided into the cover of the suture. WO 01/56626 describes a suture with spider silk and other non-spider silk element, in which the sutures can be impregnated or coated with a therapeutic substance, such as an antibacterial agent. EP 1 897 500 describes a suture with barbs and with a bioactive agent placed between barbs.

### SUMMARY OF THE INVENTION

The present invention provides a surgical suture made substantially of biological material (such as biopolymers or proteins, for example).

The invention also provides a method of suturing comprising the steps of: (i) providing a suture with a core made substantially of biological material such as biopolymers or proteins and, optionally, with a cover surrounding the core; and (ii) delivering the biological material to a surgical repair site.

Other feature and advantages of the present invention will become apparent from the following description of the invention, which refers to the accompanying drawings.

### BRIEF DESCRIPTION OF THE DRAWINGS

Figure 1 illustrates a suture and/or tape comprising a biological material, and according to an exemplary embodiment of the present invention.

Figure 2 illustrates a partial, cross-sectional view of a suture and/or tape comprising a biological material, and according to another exemplary embodiment of the present invention.

Figure 3 illustrates a schematic view of a suture and/or tape comprising a biological material, and according to another exemplary embodiment of the present invention.

### DETAILED DESCRIPTION OF THE PREFERRED EMBODIMENTS

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the spirit or scope of the present invention.

The present invention provides a surgical suture made substantially of biological material such as biopolymers or proteins.

In an exemplary embodiment only, the surgical suture may comprise a core made substantially of biological material such as biopolymers or proteins and, optionally, may be provided with a cover (for example, a braided cover) surrounding the core. The section comprising the biological material may be provided as a suture tape or as a round or oval suture, or as a combination of tape and suture. Optionally, the section comprising the biological material may be provided adjacent at least a section of suture not comprising biological material.

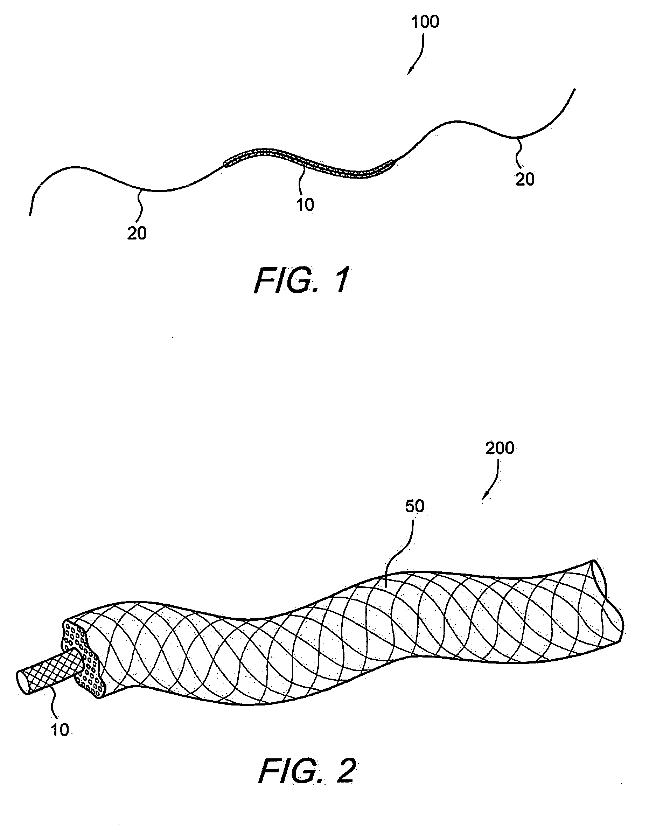

Referring now to the drawings, where like elements are designated by like reference numerals, Figures 1-3 illustrate exemplary embodiments of suture strands 100, 200, 300 of the present invention comprising a biological material such as biopolymers or proteins.

As shown in Figures 1 and 2, suture strands 100, 200 comprise at least a section or core 10 substantially formed of biological material and, optionally, at least a section or portion 20 provided adjacent the biological material of section 10. Suture section 10 may preferably comprise biological material throughout its whole length or, alternatively, may comprise biological material provided at localized areas or regions within the section 10. The biological material (for example, the collagen) may be also provided throughout the entire length and throughout the entire core of the suture, as desired.

Section 20 may also comprise biological material (which may be the same as or different from the biological material of section 10) and/or may comprise a biological material in an amount different from that of section 10. In additional embodiments, suture section 20 does not comprise biological material. Section 20 may have a diameter that is constant throughout the whole length of section 20, or may vary depending on the nature of the surgical application and of the instruments with which the suture is employed. The diameter of section 10 may be similar to or different from the diameter of section 20.

Biological material of suture section 10 may include a biopolymer (for example, a bioabsorbable polymer such as collagen or a collagen-based material, among others), or an extracellular matrix protein (such as fibronectin, elastin or laminin, among others). If collagen is employed, the collagen forming suture section 10 may be cross-linked, with additional materials, to increase the tensile strength of the collagen strands. The collagen, in its natural state or cross-linked, may be provided in the form of a plurality of collagen strips (for example, 4 inch collagen strips) that are bundle together to form the core or suture section 10 of suture 100. The collagen strips may be braided, for example, or may be provided as parallel strips adjacent to each other, to form the suture 10.

Alternatively, biological material of suture section 10 may be formed of a graft material or a combination of graft materials. In yet additional embodiments, biological material of suture section 10 is a spongy biological material which may form a scaffold or matrix containing interstitial spaces and channels that allow cellular invasion and growth (for example, fibroblast proliferation to allow tissue regeneration). The scaffold or matrix may optionally comprise additional components such as blood, proteins, growth factors or chemicals, that may be provided (by injection or impregnation, for example) within the matrix.

As such, and in accordance with additional embodiments, suture section 10 may be employed to deliver to the surgical site a biological component which includes at least one of blood, blood components or fractions, PRP, bone marrow aspirate (BMA) or autologous conditioned plasma (ACP). The biological component may be provided (by injection, impregnation or soaking, for example) directly into the suture section 10, and subsequently to the site or into the anatomical tissue, or in the vicinity of the surgical site (for example, arthroscopic site) or tissue to be repaired. In yet another embodiment, the whole length of suture 100 (including suture sections 10 and 20, or only suture section 10 if suture section 20 is absent) is soaked or injected with the biological component (for example, PRP, BMA or ACP). If desired, suture section 10 may additionally comprise components such as growth factors, additional antiseptic chemicals and/or antibiotics and/or electrolytes, or hormones or site-specific hybrid proteins (that promote or enhance the wound healing effectiveness of the growth factors), among others.

To assist in retention of the biological component by the suture 100, and if desired, at least one of suture sections 10, 20 may be coated (partially or totally) with wax (beeswax, petroleum wax, polyethylene wax, or others), silicone (Dow Coming silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, pliability, handleability or abrasion resistance, for example.

Suture section 10 may have cross-sections of various forms and geometries, including round, oval, rectangular, or flat, among others, or combination of such forms and geometries. In an exemplary embodiment only, suture section 10 may be provided as a suture tape or as a round suture, or as a combination of tape and round suture. The diameter of suture section 10 may be constant or may vary. In an exemplary embodiment, suture section 10 is about 4 to about 6 inches long and, as shown in Figure 1, is bounded at each end by suture material 20 that does not comprise biological material. In an exemplary embodiment, at least a portion of the suture (for example, about 4 to about 6 inches) comprises a section with a biological material core 10 disposed between sections 20 of the suture without biological material (for example, sections of #5 PET suture). At least one of sections 20 may contain strands of a high strength suture material, such as Arthrex FiberWire^{®} suture disclosed in U.S. Pat. No. 6,716,234, with optional colored strands to assist surgeons in distinguishing between suture lengths with the biological material and suture lengths without the biological material. If desired, at least one of the tail regions of suture sections 20 (preferably both tail regions) may have a very fine end that is coated, impregnated, or otherwise stiffened with a material such as plastic, for example.

In an exemplary embodiment only, surgical suture 100 is formed only of biological material, for example, only of collagen. Surgical suture may be formed of a core made substantially of collagen or collagen strands. The collagen may be provided as a suture tape or as a round, flat, or oval suture, or as a combination of tape and suture. The full length of the suture may include the biological material (for example, the collagen) throughout the entire length and throughout the entire core of the suture.

The biological material (for example, the collagen) may form strands and/or a matrix or sponge (i.e., collagen matrix or sponge) that allows suture section 10 to be impregnated, injected or soaked with additional components. For example, the collagen stuffed suture may be injected with at least one biological component such as blood, blood fraction, PRP, BMA or ACP, or may be soaked in a solution containing at least one biological component such as blood, blood fraction, PRP, BMA or ACP, to allow the biological component to be delivered to the surgical repair site. In an exemplary embodiment, suture material 20 (that bounds each end of collagen suture section 10) is #5 PET suture about 38 inches long, disposed at each end of the collagen section 10. The collagen section 10 advantageously delivers biological materials to the surgical repair site. If desired, the collagen stuffed suture may optionally comprise additional components such as growth factors, additional antiseptic chemicals and/or antibiotics and/or electrolytes, or hormones or site-specific hybrid proteins (that promote or enhance the wound healing effectiveness of the growth factors), among others.

Figure 2 illustrates another exemplary embodiment of suture 200 of the present invention. Suture 200 is substantially similar to suture 100 of Figure 1 but differs in that the suture section or core 10 of suture 200 is covered (partially or totally) by cover 50. In an exemplary embodiment only, suture section 10 is totally covered by cover or jacket 50.

As in the previously-described embodiment, suture section 10 may be in the form of a suture tape or a suture with the core replaced by biological material. As detailed above, suture core 10 may be formed substantially of biological material which may be optionally treated with additional materials such as blood, blood fractions, PRP, BMA, ACP, growth factors, antiseptic chemicals, antibiotics, electrolytes and various chemicals, among many others.

Cover 50 of suture 200 may be formed of suture materials, such as PET, UHMWPE, PEEK, silk nylon, and absorbable polymers, among many others. Cover 50 may also contain a bioabsorbable material, such as PLLA or one of the other polylactides, for example, and/or may be formed of twisted fibers having strands of a contrasting color added to the braided threads, to make the suture more visible during surgical procedures. The colored strands, preferably, may be dyed filaments or strands.

In exemplary embodiments, cover 50 may be a braided suture cover containing strands of a high strength suture material, such as Arthrex FiberWire^{®} suture disclosed in U.S. Pat. No. 6,716,234, with optional colored strands to assist surgeons in distinguishing between suture lengths with the biological material and suture lengths without the biological material.

As in the previously-described embodiment, at least one of cover 50 and suture section 20 may be coated (partially or totally) with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, pliability, handleability or abrasion resistance, for example.

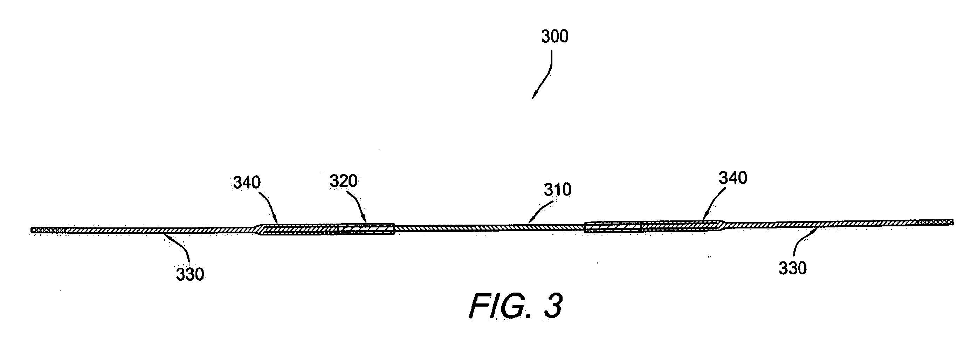

Figure 3 illustrates another exemplary embodiment of suture construct 300 comprising a biological material 310 (for example, collagen 310) provided as a strip inserted into the core of suture body 320. Strip 310 of suture construct 300 has similar characteristics as those of section or core 10 substantially formed of biological material of structures 100, 200. Suture body 320 of suture construct 300 has similar characteristics as those of section or portion 20 of structures 100, 200.

In the exemplary embodiment of Figure 3, two suture tails 330 are provided adjacent each splice 340. Suture body 320 may be formed of polyester (for example, braided polyester) and the suture tails 330 may be also formed of polyester or a similar material. Strip 310 may be formed of collagen.

In an exemplary embodiment only, suture body 320 may be formed of braided polyester with polyester core and collagen strip 310 inserted into the core of suture. Suture tails 330 may be braided polyester with a polyester core and spliced to the suture.

As in the previously-described embodiment, suture construct 300 may be coated (partially or totally) with wax (beeswax, petroleum wax, polyethylene wax, or others), silicone (Dow Coming silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, pliability, handleability or abrasion resistance, for example. For example, a coating may be provided to the yarns forming the braided suture construct 300 before braiding. Polyester yarns for the braided construct of the suture body may be coated using a silicone elastomer (or a similar material as detailed above) prior to braiding. Similarly, the suture tails may be coated using the same or different coating material after braiding and before splicing.

The suture construct of the present invention has applicability to suture applications that may be employed in surgical procedures such as rotator cuff repair, Achilles tendon repair, patellar tendon repair, ACL/PCL reconstruction, hip and shoulder reconstruction procedures, and applications for suture used in or with suture anchors. In exemplary embodiments only, the suture construct of the present invention may be employed in suture applications that do not involve knot tying, for example, for use with suture anchors (such as PushLock™ suture anchor) or for knotless arthroscopic suture repairs (such as knotless single row rotator cuff repair, or SpeedBridge™ repair using no knots and only suture passing steps), among many others.

## Claims

1. A suture strand (100, 200), comprising:
a first portion (10) comprising a core made of collagen or graft and a cover (50) surrounding the core, the cover comprising braided suture of a high strength material; and
first and second end portions (20) in contact with the first portion (10) and located at respective opposite ends of the first portion (10), the first and second end portions (20) having a different diameter than the diameter of the first portion (10).

2. The suture strand (100, 200) of claim 1, further comprising a biological component including at least one of blood, blood components, plateletrich plasma, autologous conditioned plasma and bone marrow aspirate.

3. The suture strand (100, 200) of claim 1, wherein the collagen is provided in the form of collagen strips bundled together to form the core.

4. The suture strand (100, 200) of claim 1, wherein the first portion (10) is coated with a material comprising wax, silicone, polytetrafluoroethylene, and polybutylate acid.

5. The suture strand (100, 200) of claim 1, wherein the first and second end portion (20) are coated with a plastic material.

6. The suture (100, 200) strand of claim 1, wherein the first and second end portions (20) are impregnated with a plastic material.

## Patentansprüche

1. Wundnahtfaden (100, 200) umfassend:
einen ersten Bereich (10) umfassend einen Kern, hergestellt aus Kollagen oder Körpergewebe und einer Hülle (50), welche den Kern umschließt, wobei die Hülle eine geflochtene Wundnaht aus einem hochfestem Material umfasst; und
erste und zweite Endbereiche (20), welche in Kontakt mit dem ersten Bereich (10) sind und an den jeweils gegenüberliegenden Enden des ersten Bereiches (10) lokalisiert sind, wobei die ersten und zweiten Endbereiche (20) einen anderen Durchmesser als der Durchmesser des ersten Bereiches (10) aufweisen.

2. Wundnahtfaden (100, 200) nach Anspruch 1, weiterhin umfassend eine biologische Komponente die mindestens eines von Blut, Blutkomponenten, thrombozytenreiches Plasma, autolog konditioniertes Plasma und Knochenmarkaspirat beinhaltet.

3. Wundnahtfaden (100, 200) nach Anspruch 1, wobei das Kollagen, in Form von zusammen gebündelten Kollagenstreifen, um den Kern zu bilden, bereitgestellt ist.

4. Wundnahtfaden (100, 200) nach Anspruch 1, wobei der erste Bereich (10) mit einem Material umfassend Wachs, Silikon, Polytetrafluorethylen und Polybutylatsäure beschichtet ist.

5. Wundnahtfaden (100, 200) nach Anspruch 1, wobei die ersten und zweiten Endbereiche (20) mit einem Kunststoffmaterial beschichtet sind.

6. Wundnaht (100, 200) Faden nach Anspruch 1, wobei die ersten und zweiten Endbereiche (20) mit einem Kunststoffmaterial imprägniert sind.

## Revendications

1. Fil de suture (100, 200) comprenant :
une première partie (10) comprenant une âme faite de collagène ou de greffe et une couverture (50) entourant l'âme, la couverture comprenant une suture tressée d'une matière à haute résistance mécanique ; et
des première et seconde parties extrêmes (20) en contact avec la première partie (10) et situées à des extrémités opposées respectives de la première partie (10), les première et seconde parties extrêmes (20) ayant un diamètre différent du diamètre de la première partie (10).

2. Fil de suture (100, 200) selon la revendication 1 comprenant en outre un composant biologique incluant au moins l'un du sang, des composants sanguins, du plasma riche en plaquettes, du plasma conditionné autologue et d'un produit d'aspiration de moelle osseuse.

3. Fil de suture (100, 200) selon la revendication 1 où le collagène est fourni sous forme de bandes de collagène réunies en faisceau pour former l'âme.

4. Fil de suture (100, 200) selon la revendication 1 où la première partie (10) est revêtue d'une matière comprenant une cire, un silicone, du polytétrafluoroéthylène et un acide polybutylate.

5. Fil de suture (100, 200) selon la revendication 1 où les première et seconde parties extrêmes (20) sont revêtues d'une matière plastique.

6. Fil de suture (100, 200) selon la revendication 1 où les première et seconde parties extrêmes (20) sont imprégnées d'une matière plastique.
